**Europäisches Patentamt**

**European Patent Office**

**Office européen des brevets**

(11) Veröffentlichungsnummer: **0 139 898**
**B1**

(12) **EUROPÄISCHE PATENTSCHRIFT**

(45) Veröffentlichungstag der Patentschrift: **08.08.90**

(51) Int. Cl.⁵: **G 01 N 33/00**, G 08 B 17/10

(21) Anmeldenummer: **84109040.0**

(22) Anmeldetag: **31.07.84**

(54) **Gasmessgerät.**

(30) Priorität: **12.08.83 DE 3329432**

(43) Veröffentlichungstag der Anmeldung:
**08.05.85 Patentblatt 85/19**

(45) Bekanntmachung des Hinweises auf die
Patenterteilung:
**08.08.90 Patentblatt 90/32**

(84) Benannte Vertragsstaaten:
**BE FR GB IT**

(56) Entgegenhaltungen:
**DE-A-2 714 040**
**DE-B-2 829 931**
**US-A-3 906 473**
**US-A-3 997 837**
**US-A-4 219 806**

(73) Patentinhaber: **AUERGESELLSCHAFT GMBH**
**Thiemannstrasse 1**
**D-1000 Berlin 44 (DE)**

(72) Erfinder: **Höht, Wolfgang**
**Bohm-Schuch-Weg 15**
**D-1000 Berlin 47 (DE)**

Courier Press, Leamington Spa, England.

## Beschreibung

Die Erfindung betrifft ein Gasmeßgerät der im Oberbegriff des Patentanspruchs 1 angegebenen Gattung.

Bei Gasmeßgeräten mit unabhängig voneinander einstellbaren Meßkreisen ist es bekannt, ein Abzeigeinstrument für alle Meßkreise vorzusehen, wobei die An- bzw. Umschaltung des Anzeigeinstruments an die gewünschten Meßkreise innerhalb der Meßkreise erfolgt (GB—PS 1 447 488). Durch diese Maßnahme wird die Genauigkeit der Anzeige negativ beeinflußt.

Aufgabe der Erfindung ist es, ein Gasmeßgerät der angegebenen Gattung zu schaffen, bei dem die automatische Umschaltung des eine alarmauslösende Gaskonzentration aufweisenöen Meßkreises zur Anzeige dieser Konzentration auf ein allen Meßkreisen zugehöriges Anzeigeinstrument außerhalb der die Genauigkeit der Anzeige beeinflussenden Meßkreise erfolgt, und darüberhinaus die nichtalarmauslösenden Meßwertkomponenten jederzeit wahlweise abgefragt und zur Anzeige gebracht werden können.

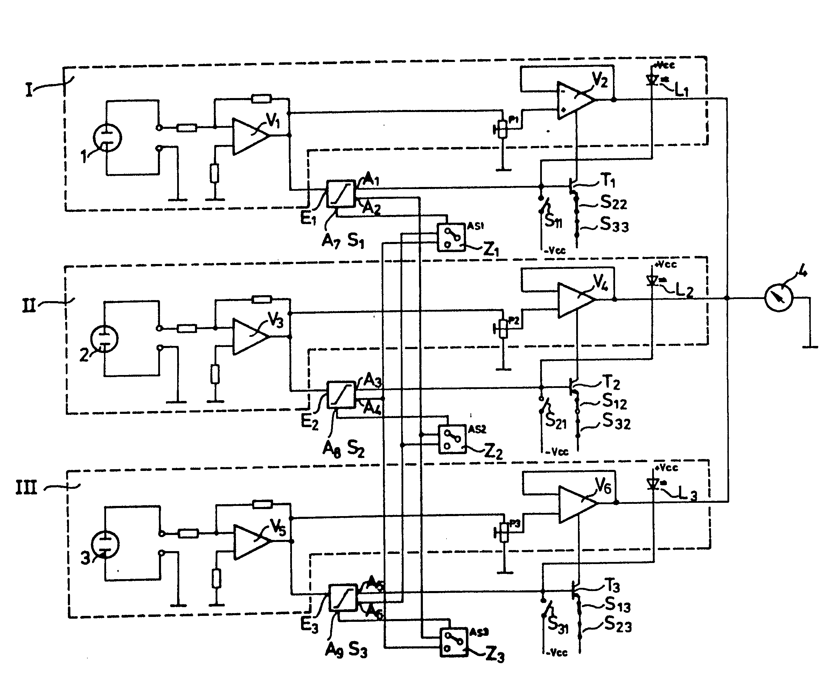

Diese Aufgabe wird erfindungsgemäß durch die kennzeichnenden Merkmale des Patentanspruchs 1 gelöst. Weitere zweckmäßige Ausgestaltungen der Erfindung sind Gegenstand von Unteransprüchen. Ein Ausführungsbeispiel der Erfindung ist in der Zeichnung dargestellt und wird im einzelnen näher beschrieben.

Die einzige Figur zeigt ein Schaltbild der Meßanordnung.

Die Meßanordnung besteht im wesentlichen aus den drei Meßkreisen I, II und III und aus einem Anzeigeinstrument 4 für alle Meßkreise. Für jeden Meßkreis sind folgende Elemente vorgesehen, die anhand des Meßkreises I beschrieben werden. Dem Gassensor 1 ist ein Signalverstärker V1 und diesem ein als Spannungsfolger geschalteter Trennverstärker V2 nachgeschaltet. Parallel zu den Verstärkern V1 und V2 ist ein Schwellwertschalter S1 und ein Transistor T1 geschaltet. Der Schaltpunkt des als elektronischer Schalter arbeitenden Verstärkers V2 wird mittels eines Potentiometers 5 eingestellt. In die Basisleitung des Transistors T1 ist ein Mehrfunktionsschalter S11 geschaltet, dessen Kontakte S12 und S13 in der Emitterleitung der Transistoren T2 und T3 liegen, die den Meßkreisen II und III zugeordnet sind. Der Mehrfunktionsschalter ist ein Schalter mit Arbeits- und Ruhekontakten oder ein elektronischer Schalter mit logischer Verknüpfung.

Die Meßkreise I, II und III sind untereinander mittels Analogschalter Z1, Z2 und Z3 schaltungstechnisch verknüpft bzw. verbunden. Die Analogschalter werden von den Schwellwertschaltern S1, S2, S3 gesteuert. Die Meßanordnung selbst kann zwei Funktionen erfüllen und zwar:

1. Überwachen und Warnen bei Erreichen einer hohen schadstoffhaltigen Gaskonzentration in der Umgebungsluft, und

2. Messen und Anzeigen der jeweils an den Meßkreisen anstehenden Gaskonzentrationen, die unterhalb einer schädlichen Konzentration liegen.

Je nach der höchsten schadstoffhaltigen Gaskonzentration aktiviert die Meßanordnung selbsttätig den Meßkreis, der dieser Konzentration zugeordnet ist, und gibt Alarm, während gleichzeitig die von einer niedrigeren Gaskonzentration beaufschlagten Meßkreise desaktiviert und somit ausgeschaltet werden.

Die alarmauslösende Meßkomponente des entsprechenden Meßkreises wird am Anzeigeinstrument 4 angezigt und eine Leichtdiode L1, L2 oder L3 kennzeichnet optisch diesen Meßkreis.

Sollen nichtalarmauslösende Meßwertkomponenten der drei Meßkreise I, II oder III gemessen werden, so erfolgt dies durch Betätigen des jedem Meßkreis zug-eordneten Mehrfunktionsschalters S11, S21 oder S31, der Schaltelemente aufweist, die einmal den abzufragenden Meßkreis für die Messung und Anzeige der anstehenden Gaskonzentration am Anzeigeinstrument 4 aktivieren und zum anderen gleichzeitig die Übrigen Meßkreise desaktivieren. Damit wird sichergestellt, daß nacheinander in beliebiger Reihenfolge Meßwerte der drei Meßkreise I, II und III abgefragt und zur Anzeige gebracht werden können.

Im einzelnen läuft folgender Vorgang ab:

Die Meßanordnung ist en eine Gleichspannung angeschlossen. Die von den Gassensoren 1, 2 und 3 abgegebenen Meßsignale werden den Verstärkern V1, V2 und V3 zugeführt, und die verstärkten Meßsignale stehen jeweils an den Eingängen E1, E2 und E3 der Schwellwertschalter S1, S2 und S3 an. Die Schaltschwellen der Schwellwertschalter S1, S2 und S3 sind auf die den alarmauslösenden Meßwerten entsprechenden Meßsignale eingestellt.

Überschreitet beispielsweise das vom Gassensor 1 kommende Meßsignal die eingestellte Schaltschwelle des Schwellwertschalters S1, so wird dessen Ausgang A1 leitend und steuert den Transistor T1 durch, der Trennverstärker V2 wird aktiviert, und das am Ausgang des Verstärkers V2 liegende Anzeigeinstrument 4 zeigt den alarmauslösenden Meßwert an, während die über den Schwellwertschalter S1 an Spannung gelegte Leuchtdiode L1 den eingeschalteten Meßkreis I anzeigt. Gleichzeitig zu diesem Vorgang wird der Ausgang A2 des Schwellwertschalters S1 leitend und steuert die Analogschalter Z2 und Z3 an, die dann die Schwellwertschalter S2 und S3 desaktivieren, so daß eine weitere Alarmauslösung und Meßwertanzeige durch die Gassensoren 2 und 3 der Meßkreise II und III nicht erfolgen kann. Falls beim Meßkreis II oder die eingestellte Schaltschwelle des Schwellwertschalters S2 des vom Gassensor 2 kommende Meßsignal überschreitet, dann wiederholt sich analog der vorstehend beschriebene Vorgang wie beim Gassensor 1 des Meßkreises I. Wenn kein Alarm ansteht, können die nichtalarmauslösenden Meßwertkomponenten wahlweise abgefragt werden. Zur Auslösung der Meßwertkomponente des Gassensors 2 beispielsweise ist die Taste des Mehrfunktionsschalters S21 zu betätigen, dessen in der Emitterleitung der Transistoren T1 und T2 angeordneten Kontakte S22 und S23 sich öffnen. Die Meßkreise I und

III sind dadurch für eine Anzeige am Anzeigeinstrument 4 gesperrt. Eine Anzeige erfolgt nur für den Meßkreis II, was auch durch die Leuchtdiode L2 optisch kenntlich gemacht wird.

**Patentansprüche**

1. Gasmeßgerät mit einer aus mehreren Meß-kreisen bestehenden Meßanordnung zum Mes-sen und Überwachen der Konzentration von Mischgasen und Dämpfen in Luft, wobei jeder Meßkreis einen Gassensor aufweist, der über einen Schwellwertschalter an ein Anzeigeinstrument für alle Meßkreise geschaltet ist, dadurch gekennzeichnet, daß

a) die Meßkreise (I, II und III) untereinander über Analogschalter (Z1, Z2 und Z3) schaltungsmäßig derart verbunden sind, daß sie eingangsseitig jeweils an einen Ausgang (A7, A8 und A9) und ausgangsseitig an einen weiteren Ausgang (A2, A4 und A6) der Schwellwertschalter (S1, S2 und S3) angeschlossen sind,

b) der Schwellwertschalter (S1, S2 oder S3) des jeweiligen alarmauslösenden Meßkreises (I, II oder III) den Analogschalter (Z1, Z2 oder Z3) ansteuert, und dieser die Schwellwertschalter der nichtalarmauslösenden Meßkreise desaktiviert,

c) ein an den Ausgang (A1, A3 oder A5) des Schwellwertschalters (S1, S2 oder S3) angeschlossener Transistor (T1, T2 oder T3) an einen als Spannungsfolger geschalteten Trennverstärker (V2, V4 oder V6) angeschlossen ist, der ausgangsseitig mit dem Anzeigeinstrument (4) verbunden ist.

2. Gasmeßgerät nach Anspruch 1, dadurch gekennzeichnet, daß in die Basisleitung des Transistors (T1, T2 oder T3) ein Mehrfunktionsschalter (S11, S21 oder S31) geschaltet ist, dessen Kontakte (S12, S13 oder S22, S23 oder S32, S33) in der Ermitterleitung der Transistoren (T1, T2 oder T3) liegen.

3. Gasmeßgerät nach Anspruch 2, dadurch gekennzeichnet, daß der Mehrfunktionsschalter (S11, S21 oder S31) ein Schalter mit Arbeits- und Ruhekontakten ist.

4. Gasmeßgerät nach Anspruch 2, dadurch gekennzeichnet, daß der Mehrfunktionsschalter ein Schalter mit logischer Verknüpfung ist.

5. Gasmeßgerät nach Anspruch 1 und 2, dadurch gekennzeichnet, daß Leuchtdioden (L1, L2 oder L3) vorgesehen sind, die den Einschaltzu-stand des jeweiligen Meßkreises (I, II oder III) anzeigen.

6. Gasmeßgerät nach Anspruch 1 und 2, dadurch gekennzeichnet, daß die elektronischen Bauelemente der Warnfunktion dem Anzeigeinstrument (4) vorgeschaltet sind.

**Revendications**

1. Appareil de mesure de gaz comprenant un système de mesure constitué de plusieurs circuits de mesure pour mesurer et surveiller la concentration de mélanges gazeux et de vapeurs dans l'air, chaque circuit de mesure présentant un détecteur de gaz connecté à travers un interrupteur à valeur de seuil à un instrument indicateur pour tous les circuits de mesure, caractérisé en ce que

a) les circuits de mesure (I, II et III) sont interconnectés par des commutateurs analogiques (Z1, Z2 et Z3) de manière qu'ils soient chacun reliés, par l'entrée, à une sortie (A7, A8 et A9) des interrupteurs à valeur de seuil (S1, S2 et S3) et, par leur sortie, à une autre sortie (A2, A4 et A6) de ces interrupteurs (S1, S2 et S3),

b) l'interrupteur à valeur de seuil (S1, S2 ou S3) du circuit de mesure (I, II ou III) déclenchant une alarme, pilote le commutateur analogique (Z1, Z2 ou Z3) et celui-ci rend inopérant les interrupteurs à valeur de seuil des circuits de mesure ne déclenchant pas d'alarme et

c) un transistor (T1, T2 ou T3), relié à la sortie (A1, A3 ou A5) de l'interrupteur à valeur de seuil (S1, S2 ou S3), est connecté à un ampli-séparateur (V2, V4 ou V6) monté comme suiveur de tension et dont la sortie est reliée à l'instrument indicateur (4).

2. Appareil de mesure de gaz selon la revendication 1, caractérisé en ce qu'un interrupteur à plusieurs fonctions (S11, S21 ou S31) est interposé dans la ligne de base du transistor (T1, T2 ou T3) et comporte des contacts (S12, S13 ou S22, S23 ou S32, S33), dans la ligne d'émetteur des transistors (T1, T2 ou T3).

3. Appareil de mesure de gaz selon la revendication 1, caractérisé en ce que l'interrupteur à plusieurs fonctions (S11, S21 ou S31) est un interrupteur possédant des contacts de travail et de repos.

4. Appareil de mesure de gaz selon la revendication 2, caractérisé en ce que l'interrupteur à plusieurs fonctions est un interrupteur à élément logique.

5. Appareil de mesure de gaz selon les revendications 1 et 2, caractérisé en ce qu'il comprend des diodes électroluminescentes (L1, L2 ou L3) qui indiquent l'état enclenché du circuit de mesure (I, II ou III) correspondant.

6. Appareil de mesure de gaz selon les revendications 1 et 2, caractérisé en ce que les composants électroniques pour la fonction d'avertissement sont prévus en amont de l'instrument indicateur (4).

**Claims**

1. A gas measuring device comprising a measuring system having several measuring circuits for measuring and monitoring the concentration of mixed gases and vapours in air, each measuring circuit comprising a gas sensor which is coupled via a threshold value switch to an indicator instrument for all the measuring circuits, characterised in that

a) the measuring circuits (I, II and III) are interconnected circuit-wise via analog switches (Z1, Z2 and Z3) in such a way that they are each connected on the input side to an input (A7, A8 and A9) and on the output side to another output (A2, A4 and A6) of the threshold value switches (S1, S2 and S3),

b) the threshold value switch (S1, S2 or S3) of the momentarily alarm-triggering measuring circuit (I, II or III) triggers the analog switch (Z1, Z2 or Z3) and the latter deactivates the threshold value switches of the measuring circuits which are not triggering an alarm,

c) a transistor (T1, T2 or T3) connected to the output (A1, A3 or A5) of the threshold value switch (S1, S2 or S3) is connected to a buffer amplifier (V2, V4 or V6) connected as a voltage follower which is connected on the output side to the indicator instrument (4).

2. A gas measuring device according to claim 1, characterised in that in the base conductor of the transistor (T1, T2 or T3) there is connected a multifunction switch (S11, S21 or S31) of which the contacts (S12, S13 or S22, S23 or S32, S33) are situated in the emitter conductor of the transistors (T1, T2 or T3).

3. A gas measuring device according to claim 3 characterised in that the multifunction switch (S11, S21 or S31) is a switch with operating and idle contacts.

4. A gas measuring device according to claim 2 characterised in that the multifunction switch is a switch with a logic connector.

5. A gas measuring device according to claims 1 and 2, characterised in that light-emitting diodes (L1, L2 or L3) are provided which indicate the switched on condition of the measuring circuit (I, II or III) in question.

6. A gas measuring device according to claims 1 and 2, characterised in that the electronic components of the alarm function are preconnected to the indicator instrument (4).

1